# EUROPEAN PATENT APPLICATION

(11) **EP 1 260 242 A1**
(43) Date of publication of application: **27.11.2002**
(21) Application number: 01909408.5
(22) Date of filing: 13.02.2001
(51) Int. Cl.: A61M 5/50

(54) **A SELF-DESTRUCTING SAFETY SYRINGE**

(30) Priority: 24.02.2000 CN 00205827
(71) Applicant: Wang, Xiping, Haidian District, Beijing city 100088 (CN)
(72) Inventor: WANG, Xiping, Haidian District, BeijingCity 100088 (CN); ZHANG, Bizhu, Beijing 100037 (CN); WANG, Xiopong, Bei jing City 100083 (CN)
(74) Representative: Reinhard - Skuhra - Weise & Partner
(86) International application number: CN0100122
(87) International publication number: WO01062321

(57) **Abstract**

The present invention relates to a self-destructive safety syringe which comprises a needle tip, a needle-tip seat, a needle barrel, a rubber piston as well as a piston push rod. The rear end of the needle-tip seat has a piercing cone possessing a bluff, the front end of the piston push rod has an elastic chuck matching with the piercing cone. The rubber piston is a hollow cylinder sleeved onto the elastic chuck, and the end face facing the piercing cone of said rubber piston has a thin film area. It is a syringe of better use safety, said syringe will not, while in use, have the manufacture defects or faults such as puffs etc. dropped into the medical fluid, entirely avoiding the serious harms to the human body because of the manufacture faults.

The syringe is easy to manufacture with lower cost and convenience in use.

## Description

### BACKGROUND OF THE INVENTION

### 1. Field of the Invention

The present invention relates to a syringe, particularly, relates to a self-destructive safety syringe, belonging to the medical therapeutical apparatus technical field.

### 2. Background Art

In the medical therapeutical trade, the once-for-all products are stressed more and more in order to avoid cross infection, and thus, the once-for-all syringe comes into operation. Up to the present, the once-for-all syringe used broadly pertains to but the administrative category; that is to say, the structure of the syringe itself does not possess the once-for-all feature, it is thrown away after being used for once according to the administrative regulation of the controlling unit. Hence, there exists the possibility for repeated uses of it, leading to potential cross infection. In addition, the used syringe is, during its subsequent disposal, extremely easy to piercingly wound the medical personnel and the patient, resulting in further infections. Therefore, many are making great efforts to research and fabricate the once-for-all syringe having safety structure.

For the time being, there are mainly two classes of safety syringes. One class of them representative of US4838869, US5324265, US5407436, CN10923251 etc., are typical design using the spring force as the automatic retracting force of the needle tip (sometimes called plunger). This sort of design reads that the needle tip (or its holder) is installed on the pressed spring. While at the finish of injecting, the push rod releases the support of the spring, so that the needle tip retracts into the hollow push rod or the needle barrel by means of the spring. This sort of safety syringe tell varieties of specific structures, their major merit, especially of certain very well designed products, is their very simplicity and convenience in use, but there exist for many of them the following problems:
1. the structure therefor is complicated, there are too many parts of them, and the assembling thereof is difficult;
2. because of the above, the production cost is rather higher and is difficult to be acceptable in marketing;
3. being restricted by the structure, the fluid outlet can hardly be designedly set at the foremost end of the needle barrel volume, leading to an increment of residue fluid and to some inconvenience to release the bubbles of the injecting fluid.

The other major class of syringes read that at the completion of the injection, the needle-tip seat fits up with the related components of the push rod as a whole; when the push rod is drawn back, the needle tip is readily retracted into the needle barrel. For this class of syringes, the structure is simpler and the production cost is lower in comparison with the former class, but there exists an un-negligible safety problem in the designs for the majority of them. For example:

The Chinese Patent CN2270509Y discloses a "back-draw safety syringe" in which there is set a mutually-matching hook-up mechanism between the push-in section of the needle-tip root and the front end of the piston rod. Thus the needle-tip and the piston rod will be readily hooked up after the injection being completed and the needle-tip can be readily pulled back into the needle barrel. However, there still exists the following defect in this kind of syringe:

The hook-up mechanism between the needle-tip and the piston rod is exposed in the medical fluid. So there is much possibility for some manufacture defects or faults such as puffs or burrs etc. of the hook-up mechanism being dropped into the medical fluid, said dropped substances, if injected into one's body together with the medical fluid, will bring forth dangerous therapeutical accident. Further, this sort of syringe does not lose its injection function after being used for once (when the hook-like part is separated from the ring after the injection; for the most, only the fixture function is disabled) and there exists the possibility for it to be reused again.

U.S. Patent, US5601534, discloses a once-for-all safety syringe of very simple structure. That said safety syringe has reliability to release the gases, keeps but a very small amount of residual medical fluid, and its barb at the second end of the needle is connected hookedly with the nipple of the solid rubber piston in a declining manner. At the finish of injection, the two are connectedhookedly, thus the plunger can be drawn back into the needle barrel. Although the hook connection mechanism of this sort of injector is not carried out in the medical fluid, yet there exist the following defects of its structure design, it is short of practical capability.
a) The fixture, which the barb at the second end of the needle (see Fig. 1A and Fig. 4 of said patent) to be deflected by the nipple of the push rod and to be connected hookedly with the nipple top, is influenced greatly by the size of the needle. When the needle is larger, the barb on the needle is difficult to be deflected and when the needle is smaller, the hook connection between the barb and the nipple top of the plunger is not reliable. Restricted by the structure, the barb at the second end of the needle and the nipple top of the push rod, while beinghookedly connected, are but one-sided contacted, hence it can bear but smaller pull force and can hardly draw the needle into the needle barrel reliably.
b) The rubber piston being solid, the barb at the second tail end of the needle ought to pierce the entire rubber piston throughout before it comes into a good fixture. This will result in a significant increase of the resistance of the push injection before the finish of the injection, and give a bad effect for use, especially when the needle is larger.
c) The injection needle, while piercing the skin, receives a push force towards the in side of the needle barrel. However, at the finish of the injection, the second end of the needle ought to overcome the outward push force from its deflection resulted by the nipple of the push rod and from piercing the solid rubber piston. When the injection needle has a larger diameter, this outward push force is rather large. In order to meet the requirement of these tow forces of different directions, the needle ought to have a better match-up with the needle barrel. However, in order that the needle, after being fixed up with the push rod piston, can be drawn into the needle barrel, it is required that the needle should not be too closely matched up with said needle barrel. Using smooth stainless steel plunger to contact the plastic needle barrel is extremely difficult to meet the above two contradictory requirements (regardless of having the adhesive there on), and it is nearly impossible, speaking from industrial production.
d) At the completion of the injection, it is required that the needle is drawn back into the needle barrel, and this requires then that the needle and the needle barrel only be slidably matched up. Because of the two being of a hard contact, assuring air-tightness turns out to be difficult. Therefore, at the draw of fluid, it is very possible for air to enter into the needle barrel along the wall matching the needle and the needle barrel. While at expelling out the fluid, it is also possible for the injection fluid to leak out along the matching portion (as a matter of cause, if some adhesive there, it could become better).
   Further, one and same type of needle barrel for this sort of syringe can only match up with one sort of needle, said one and same type of needle barrel, while in use, cannot match up with needle of different size.

### SUMMARY OF THE INVENTION

The object of the present invention is achieved as follows: A self-destructive safety syringe comprises needle tip, needle-tip seat, needle barrel, rubber piston as well as piston push rod. Said needle tip is a trocar in the needle-tip seat. Said needle-tip seat is sealedly installed at the front end of said needle barrel. Said rubber piston as well as piston push rod is installed in sequence in said needle barrel. It is characterized in that said needle-tip seat has, at its rear end, a piercing cone with a bluff and said piston push rod has, at its fore end, an elastic chuck matching with said piercing cone. Said rubber piston is a hollow cylinder which is sleeved onto the said elastic chuck and the end face facing said piercing cone of which has a thin film area. At the finish of the injection, said piercing cone can easily piercingly break the thin film area of the rubber piston and make a fixture with the elastic chuck in the rubber piston. When the piston push rod is drawn backwards, the needle-tip seat as well as the needle tip will be drawn back into the needle barrel to have the syringe self-destructed.

Said needle-tip seat is installed at a step inside the fore end of the needle barrel ,in accord with the delimited space ; on it, there is sleeved a rubber sealing ring, there is matched an inner sealing groove which is inside said needle barrel fore end ; the rear portion of said inner sealing groove is a smaller arc same as that of the sealing ring and its fore portion is a longer inclined face or a larger arc so as to realize the sealing and slidable connection between the needle tip seat and the needle barrel.

Said piston push rod further comprises a resistance plate fixedly connected with said elastic chuck, a hand push plate (in the end of the said piston push rod), and a cross rib plate weakly connected with the resistance plate. Said weakly connecting mechanism is that the horizontal rib plate of said cross rib plate is not directly connected with said resistance plate and there is set each above and below the vertical rib plate of said cross rib plate a small connecting pillar connected with said resistance plate. At the finish of the injection, when the piston push rod is drawn backwards, the weak connection can finally be broken and a real self-destruction of the syringe is embodied. This mechanism significantly increases the connection rigidity while failing to affect the breaking tensile strength at the same time, and has improved the connection stability of the push rod as well as the production yield.

One rib of said cross rib plate has a saw-indented groove, and a thin film on the wing plate of the needle barrel tail is connected with a check plate having a shallow groove. It forms a simple lock-up mechanism of the syringe which may prevent the syringe from being self-destructed caused by misleading operation of it before use.

The bottom face of said piercing cone is not vertical to the axial line. This tells that after the needle tip is drawn back into the needle barrel, the needle tip point turns out to be very close to the inner wall of the needle barrel and not easy to plunge out of the needle barrel once more.

The present invention is advantageous over the conventional syringes in that:
a). The fixture between the elastic chuck and the piercing cone is embodied in the rubber piston of good air-tight sealing, therefore the possibility of the manufacture faults (such as puffs or burrs) dropping into the medical fluid is completely avoided during the fixing from the elastic chuck to the piercing cone. Such reasonable structure improves the use safety very greatly and gives the said syringe very nice practical capability, avoiding the insufficiency of the prior art. They are specifically shown in the following aspects:
   a)-1. The fixture is achived by the piercing cone and the elastic chuck which is located inside the rubber piston. Both the piercing cone and the elastic chuck are made of harder plastics leads to the loosening-resistant force being much larger than the motion resistance between of the sealing ring on the needle-tip seat and the needle barrel sealing groove. This sort of fixture is very reliable, therefore, the needle-tip seat and the needle tip can be assured to be drawn together into the needle barrel when the push rod is drawn backwards.
   a)-2 The fore end face of the rubber piston has a thin film area, thus the piercing cone portion with a smaller conic angle is very easy to pierce said area so as to make a fixture with the elastic chuck, and therefore, there will not be a significant increase of the push-injection resistance to affect the use thereof.
   a)-3 The configuration of the needle-tip seat rubber ring and a special design of the needle barrel fore end sealing groove, in addition to the step localized matching between of the needle-tip seat and the needle barrel, can fully meet the requiring conditions of the various acted forces during the use thereof, therefore, the contradictory problem relating to the matching between of the plunger and the needle barrel in Patent US 5601534 has been solved really.
   a)-4 The needle-tip seat uses a rubber ring sealing with the needle barrel, said sealing is of high reliability, no leakage will take place.
b) Said syringe has a reasonable design, nice functions, a simpler structure and a lower production cost, and is suitable for mass production. Its needle-tip seat may match with needle tips of various sizes, and turns out to be very convenient for use. Further, its needle-tip seat is adaptable for use for many syringes of different specifications, therefore the production cost can be greatly reduced.
c) In addition, the weakly connecting mechanism between the resistance plate and the cross rib plate raises significantly the connecting regidlity while not affecting the breaking tensile strength, and improves the push rod connecting stability as well as the production yield. The weakly connecting mechanism would be broken after being used and result in the syringe really to be destructed.
d) The approach that the bottom face of the piercing cone is not vertical to the axial line results in the fact that the needle tip deviates from the axial line and becomes inclined after being drawn back into the needle barrel. So the needle tip, while the push rod being pushed, will be pressed against the inner portion of the needle barrel to be bent and destructed.
e) The matching design for the groove on the cross rib plate and the check plate on the wing plate of the needle barrel tail tells that the syringe itself has possessed a safe lock-up device of simple structure and convenient operation, which may prevent the syringe from being self-destructed due to a misleading operation before use and fully avoids unnecessary extravagance or waste A brief explanation on the attached drawings

A further detailed explanation is given on the present invention in combination with the attached drawings as follows.

### BRIEF DESCRIPTION OF THE DRAWINGS

Fig.1 is a general section view of the present invention showing its structures assembled up and before use.
Fig.2 is a layout for the structures of the present invention after its use with the needle tip withdrawn back into the needle barrel.
Fig.3 is a layout for the structure of the present invention after its use with the needle tip there of being pressed ahead to be bent and self-destructed.
Fig.4 is a layout for the structure of the present invention with its push rod weak area being broken and self-destructed while being drawn backwards.
Fig 5 is a three-dimentional graph of the needle-tip seat for the present invention.
Fig 6 is a magnified section layout of the needle barrel front end of the present invention.
Fig 7 is a locally magnified section view of the piston push rod front end for the present invention.
Fig 8 is a section view of the piston push rod weak area.
Fig 9 is an over view of Fig,8.
Fig 10 is a section layout for the structure of the rubber piston for the present invention.

### DETAILED DESCRIPTION OF THE PREFERRED EMBODIMENT

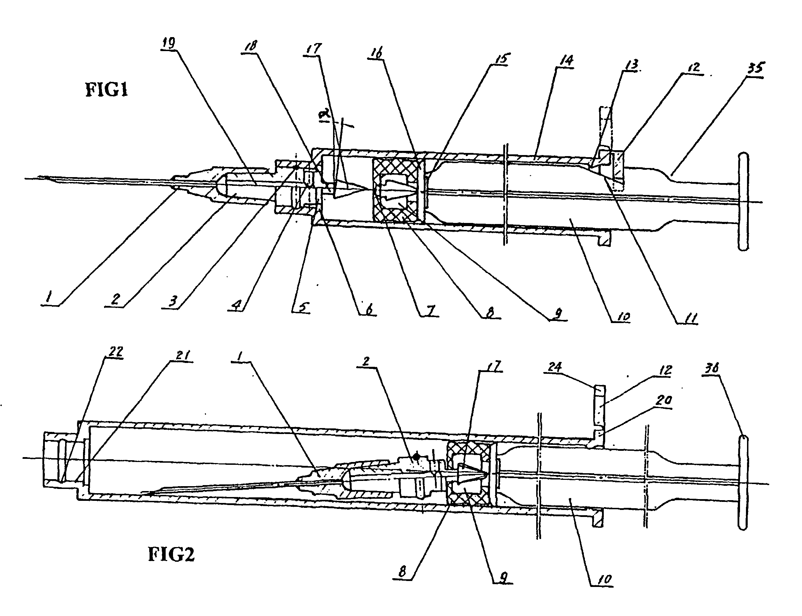

As shown in Fig 1 and Fig 2, the present invention provides a self-destructive safety syringe. It consists of a needle barrel 14, needle-tip seat 2 as well as a needle tip 1, rubber piston 8, and piston push rod 10.

The needle barrel 14 is a cylinder with the front end of which is a matching section 21 of the needle-tip seat. The diameter of said section is smaller than the diameter of the section inside the needle barrel itself. There is an inner sealing groove 22 in said matching section 21. The root of the matching section 21 in said needle barrel 14 has a step 6. The rear end of said needle barrel 14 has a wing plate 20 on which there is a check plate 12 connected by a thin film and having a shallow groove 24 at it's outer end. At the rear portion inside said needle barrel 14, there is a resistance ring 13 having a saw-indented cross section 13. The shorter side of the cross section 13 is facing the fore portion of needle barrel 14 and has a larger angle with the axial line, e.g., around 60 degrees. The longer side of the cross section 13 is facing the tail of needle barrel 14 and has a smaller angle with the axial line, e.g., around 10 degrees.

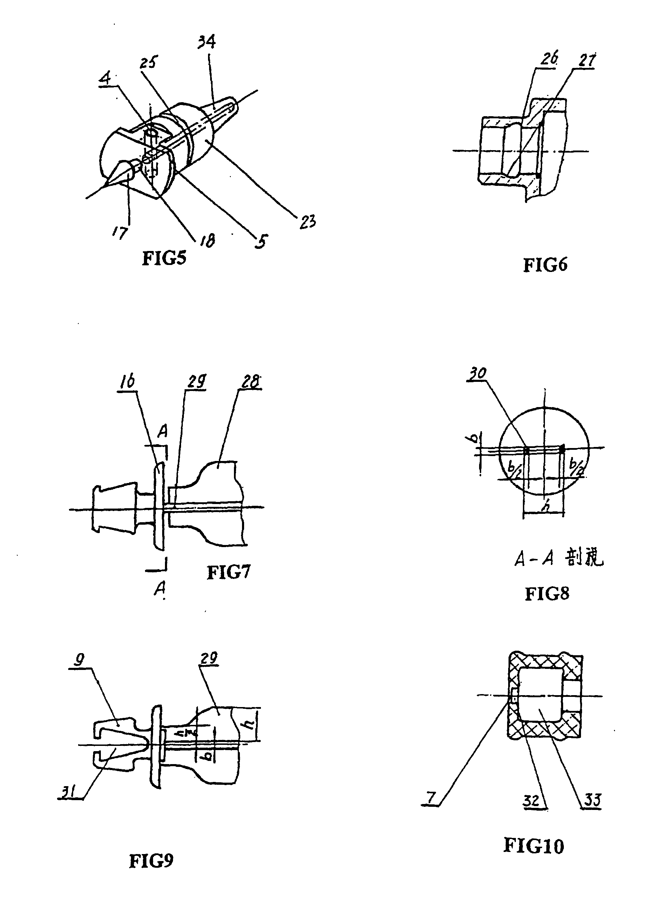

Said needle-tip seat 2, as shown in Fig. 5, has a central fluid through-hole 19 at its centre, its front end is a cone 34 capable of closely matching and connecting with the needle tip, so as to replace conveniently the needle tip of different sizes. Its medium portion is a cylinder 23 matching with the needle-tip seat matching-section 21 of said needle barrel 14. The cylinder 23 has an outer sealing groove 25 on which there is sleeved a rubber sealing ring 3 being matched with said inner sealing groove 22, thus a closer sealing is formed between needle-tip seat 2 and needle barrel 14. The rear portion 26 of said inner sealing groove 22 and the said sealing ring 3 are prepared to be of the same arc shape, as is shown in Fig 6, and the fore portion 27 thereof is prepared to be a longer inclined face or a larger arc. Thus, not only prevent the needle-tip seat 2 moving toward needle barrel 14 while fluid drainning or piercing thereto, but also can be drawn into needle barrel 14 by push rod 10 after fixture made between cone 17 and elastic chuck 9. Further, it can be more conveniently produced and refined. The rear portion of the cylinder 23 of the needle-tip seat 2 has a step 5 and is making a shape of flat and circular. There is a vertical hole 4 in the step 5 in communication with a central fluid through-hole 19 forming a medical fluid passage. Said vertical hole 4 is located at the highest position of the space accommodating the injection fluid of said syringe so that the air bubble can be easily expelled out. Said step 5 is matched with said step 6 inside said needle barrel 14 so as to prevent needle-tip seat 2 from being pushed out of needle barrel 14 and so as to reduce the injection residue. The tail of said needle-tip seat 2 is a piercing cone 17 having a bluff 18. The bluff 18 is a smooth cylinder, the piercing cone 17 is a solid pointed cone, smooth and of a smaller conic angle, e.g., 30 degrees. The bottom face of the cone 17 is made to be inclined, e.g., making a tipping angle of around 5 degrees with the vertical face.

Said rubber piston 8 as shown in Fig. 10 is a cylinder having a cavity 33. The centre of its front end face is prepared a thin film area 7 as well as a cylindrical hole 32, the diameter of which is same as or slightly smaller than that of said cone bluff 18. The thin film area 7 can be easily pierced and broken by said cone 17 at the end of the injection while no significant push-injection resistance will be resulted. The above-mentioned structure feature as well as the rubber sealing action can assure the medical fluid keeping a normal injection without back flow or with but an extremely small back flow.

Said piston push rod 10 consists of a elastic chuck 9, resistance plate 16,cross rib plate 35 as well as a hand push plate 36. Said hand push plate 36 is located at the rear end of the piston push rod 10 and is connected with the rear end of the cross rib plate 35. Said cross rib plate 35 comprises a horizontal rib plate 28 and a vertical rib plate 29, the front end of which is weakly connected with the rear end face of said resistance plate 16, as shown in Fig. 7 and Fig .8 and Fig. 9. Said weak connection means specifically: said horizontal rib plate 28 is not connected with said resistance plate 16, the gap between them is very small, e.g., 0.2∼0.5mm; the front end of said vertical rib plate 29 becomes gradually narrower, small connecting pillars 30 each at the position half the width of the rib plate 29 are connected with said resistance plate 16 and the width of the small connecting pillars 30 can be taken as around half of the rib plate 29 width. This structure significantly raises the connecting rigidity and improves the connecting stability of the push rod as well as the production yield, while not affecting the breaking tensile strength. Said vertical rib plate 29 is on its one side prepared a saw-indented groove 11 the straight side of which goes ahead, the inclined side of which goes behind and the depth of which is around half of the rib plate width. Said resistance plate 16 has a transitional arc brim on its front end face, and its rear end face brim is a straight line, its outer diameter is equal to or slightly smaller than the inner diameter of said needle barrel 14, e.g., around 0.1mm smaller than the inner diameter of the needle barrel 14, but larger than the inner diameter of said resistance ring 13, e.g., larger than 0.3∼0.6mm. Said elastic chuck 9 is connected with the front end face of said resistance plate 16, its inner cavity 31 has a shape capable of being fixed up to said cone 17, said inner cavity 31 is slightly larger than cone 17.

During the assembling, as shown in Fig.1, said needle tip 1 is sleeved onto said needle-tip seat 2, the needle-tip seat 2 is set to the matching section 21 of the needle barrel 14. Because there is said step 5 on the needle-tip seat 2, said step 7 just fits up into the fore end step 6 of the needle barrel 14 while being assembled, therefore, the needle-tip seat 2 is assured to be not expelled out of the needle barrel 14 and to be capable of supporting the injection pressure during the injection and supporting the resistance from the fixturing of the elastic chuck 9 and the cone 17 piercing the thin film area 7 at the injection finish, while not moving ahead any more. The elastic chuck 9 at the front end of the piston push rod 10 is sleeved into the inside cavity 33 of said rubber piston 8, then the two are set into the said needle barrel 14. The check plate 12 connected by the thin film onto the needle barrel 14 is turned downwards, its check groove 24 is just checked up to the vertical rib plate 29 at the saw-indented groove 11. Now, the piston push rod 10 can not move ahead, that is, a safety distance is formed at its front end so as to avoid unexpected accident.

For the use of the present invention, it is only required that the piston push rod 10 be pulled backward, then the rubber piston 8 will move backward under the action of piston push rod 10 and the medical fluid or the blood will be absorbed into. In this course, the check plate 12 will leave the rib plate 29, no longer hindering the movement of the push rod 10 because of the action of the inclined face of groove 11 and the elasticity of the thin film 7. Thus the syringe can readily fulfill normally, the drain and the push-injection functions of medical fluid or blood. When the needle tip point pierces the skin (muscle, vein etc), the piercing force will push needle-tip seat 2 to move into the needle barrel 14. But it will stop moving any more because the rubber sealing ring 3 sleeved onto the needle-tip seal 2 is limited by the rear portion arc of sealing groove 22 in the fore end hole 21 of the needle barrel 14 (changing the size of the sealing ring may obtain different motion resistance). After finishing the injection as shown in Fig, 2, the cone 17 at the rear end of said needle-tip seat 2 has pierced and broken the thin film area 7 of the rubber piston 8 and has wedged into the elastic chuck 9 of the piston push rod 10.

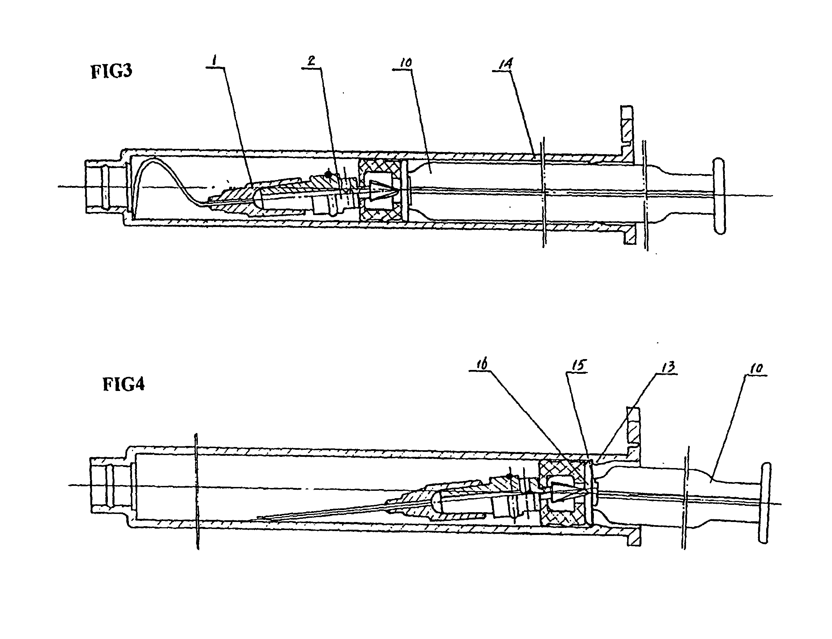

Because both the piercing cone 17 and the elastic chuck 9 are made ofharder plastics, therefore their loosening force therein between can be far larger than the motion resistance between the sealing ring 3 on the needle-tip seat and the sealing groove 22 of the needle barrel. Hence when the push rod 10 is pulled back, the needle-tip seat 2 and needle tip 1 will be pulled together into the needle barrel 14. Because of the inclined angle at the bottom face of the cone, the needle-tip 1 readily deviates the axial line and becomes inclined after being pulled into the needle barrel 14. When the piston push rod 10 keeps to be pulled out, the straight line side of the resistance plate 16 is obstructed by the shorter side of resistance ring 13. So the resistance is raised and the cross push rod 35 is separated from resistance plate 16 and destructed at the weak area 15, as shown in Fig. 4. If push ahead the piston push rod 10 again, the needle tip will press against the step 6 in the bottom of the needle barrel 14 not to continuous moving ahead, result in that the needle tip is bent and destructed by pushing ahead the piston push rod 10 continuously.

Because of the structure feature of the resistance plate 16 and the resistance ring 13, the resistance plate 16 is made to be forced to pass the resistance ring 13 and enter the needle barrel 14 under the action of the assembling force during the initial assembling. This has nothing to do with the use of said syringe. Further, when the syringe completes the drain of the medical fluid or the blood, the rubber piston 8 will not be pulled out of the needle barred 14 and the use safety will be then improved.

The present syringe can be easily produced and processed and has a higher product yield. Because of its special design in structure, it possesses much better use safety and much nicer applicable capability. It may be broadly applied to medical and therapeutical trade.

It will apparent to those skilled in the art have various modifications and various can be made in the present invention without departing from the scope or spirit of the invention. Other embodiments of the invention disclosed herein. It is intended that the specification and examples be considered as exemplary only, with a true scope and spirit of the invention being indicated by the following claims.

## Claims

1. A self-destructive safety syringe comprising a needle tip, a needle-tip seat, a needle barrel, a rubber piston as well as a piston push rod; said needle tip is sleeved onto the needle-tip seat, said needle-tip seat issealingly installed onto the front end of said needle barrel, said rubber piston as well as the piston push rod is sequentially installed into the said needle barrel, **characterized in that**:
at its rear end, said needle-tip seat has a piercing cone with a bluff; at its front end, said piston push rod has an elastic chuck matching with said piercing cone; said rubber piston is a hollow cylinder sleeved onto said elastic chuck and has a thin film area on its end face facing said piercing cone; at the finish of the injection, said piercing cone can easily piercingly break the thin film area of the rubber piston and make a fixture with the elastic chuck in the rubber piston; when the piston push rod is drawn backwards, the needle-tip seat as well as the needle tip will be drawn back into the needle barrel to have the syringe self-destructed.

2. The self-destructive safety syringe as indicated in claim 1, wherein a sealing ring is sleeved onto said needle-tip seat, there is an inner sealing groove matching with said sealing inside the front end of said needle barrel, the rear portion of said inner sealing groove is a smaller arc same as that of the sealing ring and its fore portion is a longer inclined face or a larger arc so as to realize the sealing and slidable connection between the needle tip seat and the needle barrel.

3. The self-destructive safety syringe as indicated in claim 1, wherein said piston push rod further comprises a resistance plate fixedly connected with said elastic chuck, a cross rib plate weakly connected with the resistance plate, said weakly connected structure reads that the horizontal rib plate is not connected with the resistance plate, the small pillars each above and below the vertical rib plate are not connected with said resistance plate; at the finish of the injection, when the piston push rod is drawn backwards, the weak connection can finally be broken and a real self-destruction of the syringe is embodied; this mechanism significantly increases the connection rigidity while failing to affect the breaking tensile strength at the same time, and has improved the connection stability of the push rod as well as the production yield.

4. The self-destructive safety syringe as indicated in claim 1, wherein one rib of said cross rib plate has a saw-indented groove, the thin film on the wing plate of the tail of the said needle barrel has connected with a check plate having a shallow groove; it makes up a simple lock-up mechanism of the syringe which may prevent the syringe from being self-destructed because of misleading operation of it before use.

5. A self-destructive safety syringe as indicated in claim 1, wherein the bottom face of said piercing cone is not vertical to the axial line, telling that after the needle tip is drawn back into the needle barrel, the needle tip point turns out to be very close to the inner wall of the needle barrel and not easy to plunge out of the needle barrel once more.
